# FASCICULE DE BREVET EUROPEEN

(11) **EP 1 436 037 B1**
(45) Date de publication et mention de la délivrance du brevet: **20.04.2005**
(21) Numéro de dépôt: 02772461.6
(22) Date de dépôt: 26.07.2002
(51) Int. Cl.: A61M 39/22, A61M 25/01, A61B 17/34

(54) **DISPOSITIF D'OBTURATION SELECTIVE DE L'ACCES A L'INTERIEUR D'UN CATHETER**
Vorrichtung für den selektiven Verschluss in einem Katheter
DEVICE FOR SELECTIVE ACCESS CLOSURE INSIDE A CATHETER

(30) Priorité: 11.09.2001 FR 0111750
(43) Date de publication de la demande: 14.07.2004
(73) Titulaire: SEDAT, 69540 Irigny (Rhône) (FR)
(72) Inventeur: DENOLLY, Pascal, F-38200 Jardin (FR)
(74) Mandataire: Blot, Philippe Robert Emile
(86) Numéro de dépôt international: PCT/FR2002/002692
(87) Numéro de publication internationale: WO 2003/022351

(56) Documents cités:
- DE-A- 4 213 691
- US-A- 5 195 980
- US-A- 5 325 868
- US-A- 5 423 331
- US-A- 5 685 854
- US-A- 5 827 202

## Description

La présente invention concerne un dispositif d'obturation sélective de l'accès à l'intérieur d'un cathéter, du type comportant, d'une part, un corps délimitant un conduit présentant une extrémité arrière d'introduction d'un instrument médical et une extrémité avant munie des moyens de connexion au cathéter et, d'autre part, un mécanisme de réglage de la section de passage offerte dans le conduit, lequel mécanisme de réglage comporte un organe de commutation du dispositif mobile par rapport au corps suivant une course d'ouverture pour assurer la commutation vers un état ouvert, dans lequel une section de passage nominale est offerte dans le conduit, et suivant une course de fermeture pour assurer la commutation vers un état fermé, dans lequel le passage au travers du conduit est au moins partiellement obturé.

Un tel dispositif est utilisé notamment lors des interventions d'angioplastie réalisées en cardiologie. Ces interventions consistent à conformer un vaisseau sanguin (veine ou artère) ayant subi un rétrécissement engendrant une diminution du débit sanguin. Cette conformation du vaisseau est assurée soit par une simple dilatation du vaisseau à l'aide d'un ballonnet gonflable, soit par mise en place d'une endoprothèse tubulaire auto-expansive, couramment appelée "stent".

Quel que soit le mode d'intervention, il convient d'intervenir à l'intérieur du réseau sanguin du patient. A cet effet, une incision est réalisée dans une artère, généralement l'artère fémorale, et un cathéter y est introduit. Les différents instruments nécessaires pour le traitement du vaisseau sont acheminés jusqu'au lieu d'intervention en circulant dans le réseau sanguin après avoir été introduits au travers du cathéter engagé dans l'artère fémorale. Plus précisément, un guide chirurgical constitué d'un long fil métallique souple, un ballonnet, un stent ou un autre cathéter peuvent être introduits dans le réseau sanguin au travers du cathéter pré-installé.

Afin d'éviter des saignements excessifs du patient, le cathéter engagé dans l'artère fémorale est muni, à son extrémité libre, d'un dispositif d'obturation sélective permettant d'éviter les écoulements sanguins, tout en permettant d'engager dans le cathéter les différents instruments nécessaires à l'intervention.

Des dispositifs d'obturation sélective sont déjà connus, comme US 5 195 980 et US 5 685 894. Ils comportent généralement un diaphragme obturant le passage ménagé dans le corps du dispositif. Ce diaphragme est constitué par exemple d'un joint élastique. Le dispositif comporte un mécanisme adapté pour provoquer une ouverture volontaire du joint afin de permettre l'engagement des instruments nécessaires à l'intervention. Ce mécanisme comporte généralement un organe tubulaire coulissant, déplaçable entre une position écartée du joint et une position engagée à l'intérieur du joint augmentant ainsi sa section interne. L'organe tubulaire est par exemple déplaçable en translation sous la commande d'une bague rotative ou d'un curseur coulissant. La bague ou le curseur est déplacé, dans un sens, pour provoquer l'ouverture du dispositif et, dans l'autre sens, pour provoquer sa fermeture.

Le maniement d'un tel dispositif est incommode, puisque le praticien doit utiliser ses deux mains, l'une pour maintenir le corps du dispositif et l'autre pour déplacer la bague ou le curseur.

L'invention a pour but de proposer un dispositif d'obturation sélective d'un maniement facile.

A cet effet, l'invention a pour objet un dispositif d'obturation sélective du type précité, caractérisé en ce que le sens et la direction de déplacement de l'organe de commutation suivant ladite course de fermeture, d'une part, et le sens et la direction de déplacement de l'organe de commutation suivant ladite course d'ouverture, d'autre part, sont identiques.

Suivant des modes particuliers de réalisation, le dispositif comporte l'une ou plusieurs des caractéristiques suivantes :
- la direction de déplacement de l'organe de commutation suivant lesdites courses d'ouverture et de fermeture est décalée angulairement par rapport à l'axe du conduit ;
- ledit mécanisme de réglage de la section de passage offerte dans le conduit comporte un diaphragme et une canule montée mobile dans le conduit entre une position écartée du diaphragme correspondant à l'état fermé du dispositif, et une position engagée au travers du diaphragme, correspondant à l'état ouvert du dispositif, et le mécanisme comporte des moyens de déplacement de la canule alternativement dans un sens et dans l'autre entre ses deux positions, sous la commande de l'organe de commutation, seulement lorsque l'organe de commutation est déplacé, suivant lesdites courses d'ouverture et de fermeture, dans un sens de commande identique pour les deux sens de déplacement de la canule ;
- le conduit est rectiligne, et ledit organe de commutation est déplaçable en translation suivant lesdites courses d'ouverture et de fermeture parallèlement à l'axe dudit conduit ;
- le mécanisme de réglage de la section de passage offerte dans le conduit comporte, d'une part, un coulisseau dont la canule est solidaire, lequel coulisseau est déplaçable à coulissement par rapport au corps sous la commande dudit organe de commutation et, d'autre part, une roue à rochet montée rotative par rapport au coulisseau et fixe axialement par rapport à celui-ci, le corps comportant des profils d'engrènement de la roue à rochet pour son déplacement angulaire lors du déplacement à coulissement du coulisseau, et des profils de guidage de la roue à rochet pour son guidage lors du relâchement dudit organe de commutation vers au moins deux positions axiales distinctes correspondant aux états ouvert et fermé du dispositif ;
- ledit mécanisme de commutation comporte un organe de rappel élastique de l'organe de commutation suivant lesdites courses d'ouverture et de fermeture dans le sens opposé à celui produisant la commutation du dispositif ;
- le corps présente une forme générale ovoïdale aplatie présentant deux surfaces principales opposées et une surface latérale périphérique reliant lesdites surfaces principales opposées ;
- le corps présente, sur sa surface latérale périphérique, au moins deux creux adjacents formant des surfaces d'appui parmi les doigts d'une main ;
- deux creux formant des surfaces d'appui pour les doigts d'une main sont disposés successivement suivant une direction sensiblement parallèle à l'axe du conduit ;
- ledit organe de commande fait saillie hors du boîtier entre deux creux formant des surfaces d'appui pour les doigts d'une main et l'extrémité arrière du conduit ; et
- il comporte des moyens de maintien du dispositif dans son état ouvert et dans son état fermé.

L'invention sera mieux comprise à la lecture de la description qui va suivre, donnée uniquement à titre d'exemple et faite en se référant aux dessins, sur lesquels :
- la figure 1 est une vue en élévation du dispositif d'obturation sélective selon l'invention ;
- la figure 2 est une vue en perspective éclatée du dispositif de la figure 1 ;
- les figures 3A et 3B sont des vues en perspective, respectivement, de la demi-coque principale et de la demi-coque complémentaire du boîtier du dispositif des figures 1 et 2, ces coques étant représentées avec leur face interne visible ;
- les figures 4A et 4B sont des vues en perspective d'une même roue à rochet vue suivant deux bouts opposés ;
- les figures 5A et 5B sont des vues à plus grande échelle d'un détail, respectivement, des demi-coques principale et complémentaire du boîtier du dispositif des figures 1 et 2 ;
- les figures 6, 7, 8 et 9 sont des vues en coupe longitudinale du dispositif selon l'invention à des stades différents de fonctionnement ; et
- les figures 10, 11 et 12 sont des vues en élévation du dispositif maintenu dans la main d'un praticien suivant plusieurs modes de prise en main différents.

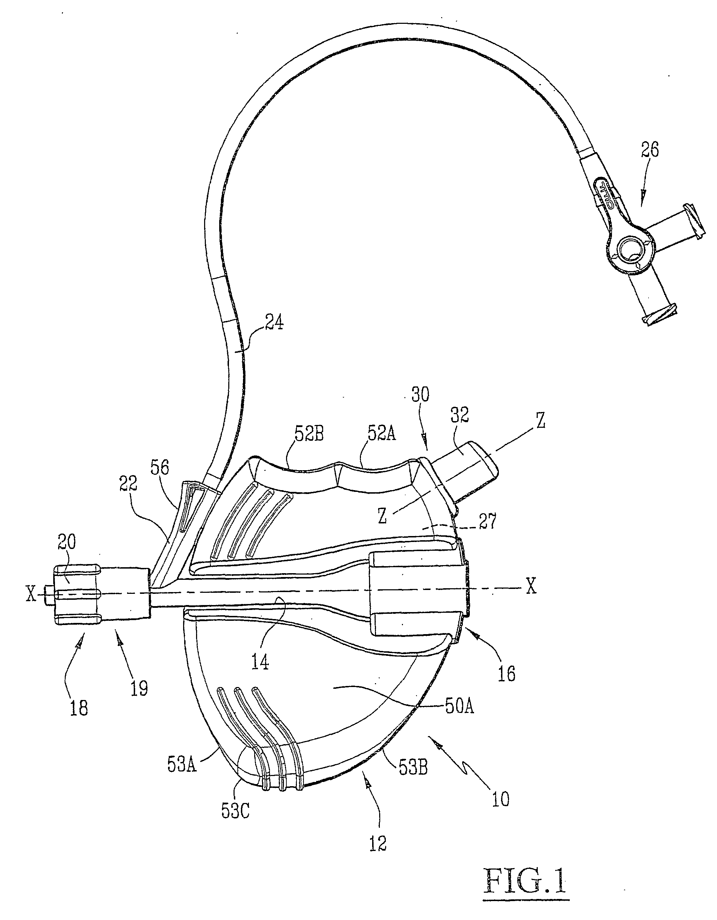

Le dispositif d'obturation sélective 10 est représenté sur la figure 1 posé à plat sur une surface. Ce dispositif comporte un corps 12 délimitant un conduit transversal 14 de passage d'un instrument filiforme destiné à être introduit dans le corps humain. Le corps 12 présente la forme générale d'une olive aplatie permettant sa prise en mains.

Le conduit 14 est rectiligne d'axe X-X. Il traverse le corps 12 de part en part et débouche à ses deux extrémités. Le conduit 14 présente une extrémité arrière 16 d'introduction des instruments filiformes de traitement. A son extrémité avant notée 18, le conduit 14 comporte des moyens 19 de liaison pour un cathéter prolongeant le conduit. Ces moyens de liaison comportent par exemple une bague rotative 20 propre à coopérer avec un embout complémentaire prévu à l'extrémité du cathéter.

Dans le mode de réalisation illustré, un piquage 22 débouche dans le conduit 14 au voisinage de l'extrémité 18 de liaison du cathéter. Ce piquage 22 est prolongé par un cathéter auxiliaire 24 dont l'extrémité libre est équipée d'un robinet trois voies 26 permettant l'introduction ou le prélèvement d'un fluide dans le cathéter quel que soit l'état d'obturation du dispositif.

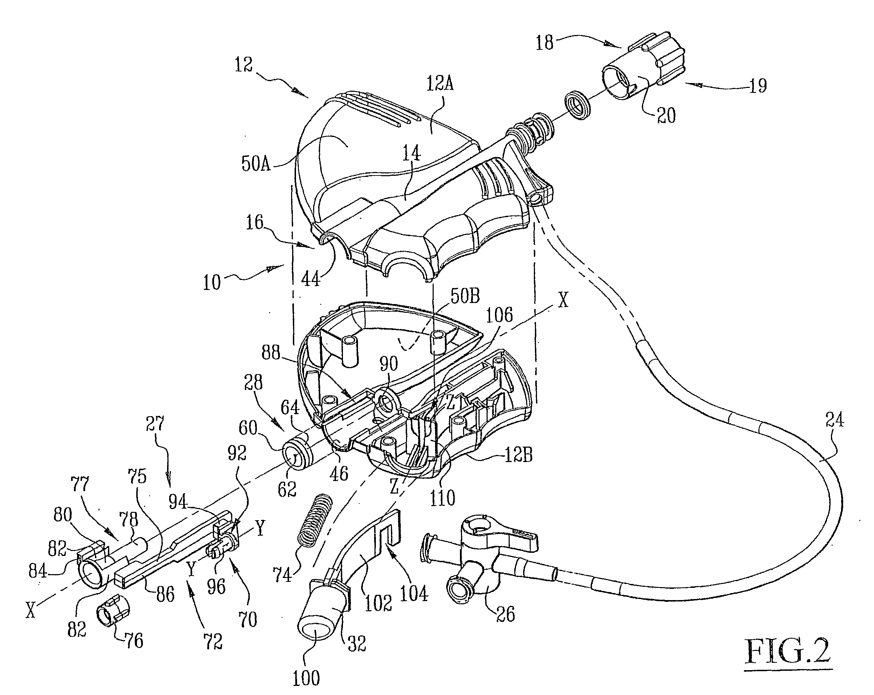

Le dispositif 10 comporte un mécanisme 27 de réglage de la section de passage offerte au travers du conduit 14. Ce mécanisme comprend, dans le conduit 14, des moyens 28 d'obturation sélective du conduit. Ceux-ci sont visibles sur la figure 2. En outre, le mécanisme de réglage 27 comprend un mécanisme 30 de commutation adapté pour une commande des moyens d'obturation 28 afin d'assurer leur commutation entre un état ouvert dans lequel une section de passage nominale est offerte au travers du conduit 14, et un état fermé dans lequel le passage au travers du conduit 14 est au moins partiellement obturé.

Ce mécanisme de commutation 30 comporte un organe de commutation 32 constitué par exemple d'un bouton poussoir déplaçable suivant une course d'ouverture pour assurer la commutation vers l'état ouvert et une course de fermeture pour assurer la commutation vers l'état fermé.

Selon l'invention, le sens et la direction de déplacement de l'organe de commutation suivant la course de fermeture, d'une part, et le sens et la direction de déplacement de l'organe de commutation suivant la course d'ouverture, d'autre part, sont identiques. En particulier, pour obtenir l'ouverture ou la fermeture du dispositif, l'organe de commutation 32 est chaque fois enfoncé dans le corps 12 du dispositif.

Le dispositif, et notamment ses éléments constitutifs vont être maintenant décrits plus en détail en regard de la figure 2 et des figures suivantes.

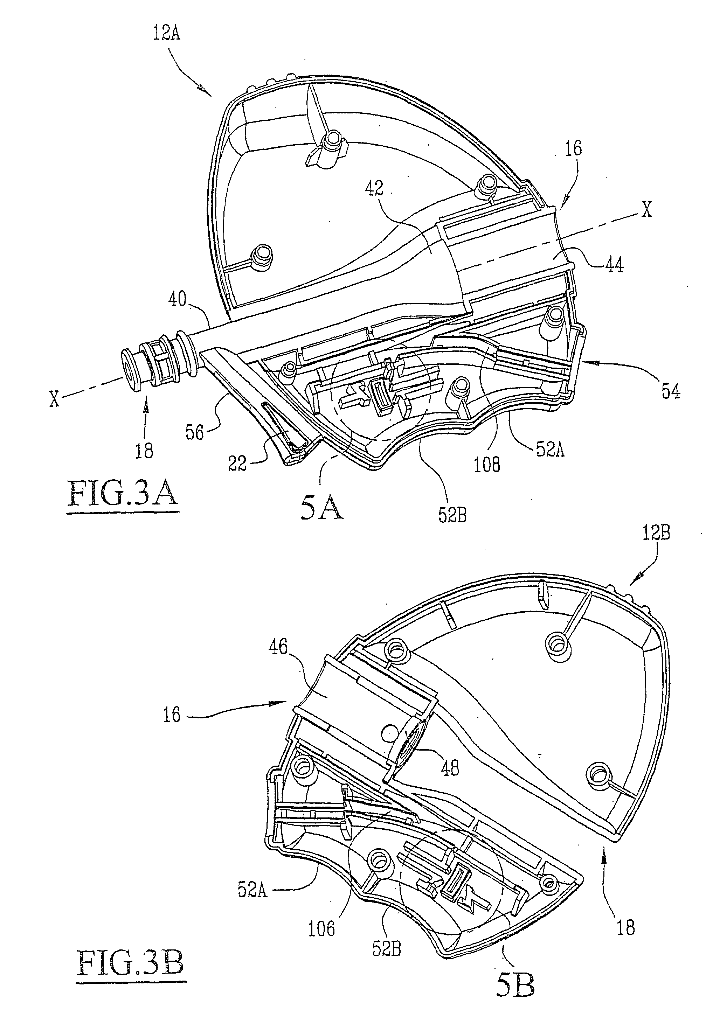

Le corps 12 est constitué de deux demi-coques 12A, 12B liées l'une à l'autre suivant un plan longitudinal médian. La première demi-coque 12A, constituant une demi-coque principale, est représentée seule sur la figure 3. Cette demi-coque est formée d'une seule pièce en matière plastique. Elle présente, dans sa partie médiane, un tronçon tubulaire 40 d'axe X-X délimitant le conduit 14 sur une partie de sa longueur, et notamment sa partie située du côté de l'extrémité avant 18. Ce tronçon tubulaire 40 présente, dans la partie médiane de la demi-coque, un tronçon divergeant de section progressivement croissante 42 en direction de l'extrémité arrière 16 du conduit. Ce tronçon divergent 32 est prolongé axialement d'un tronçon semi-cylindrique 44 propre à délimiter la moitié du prolongement du conduit 14.

La demi-coque complémentaire 12B, représentée seule sur la figure 3B, présente un tronçon semi-cylindrique 46 complémentaire du tronçon semi-cylindrique 44 adapté pour délimiter le conduit 14 dans la région de l'extrémité arrière 16. Dans la partie médiane, le conduit 14 présente un siège 48 de retenu des moyens d'obturation 28. Ce siège est venu de matière avec la demi-coque complémentaire 12B à l'extrémité du tronçon semi-cylindrique 46.

Le corps 12 présente une forme générale ovoïdale aplatie. Il présente deux surfaces principales opposées 50A, 50B s'étendant généralement parallèlement l'une à l'autre et définies respectivement par les deux demi-coques 12A, 12B.

La surface 50B définie par la demi-coque 12B est généralement plane. La surface 50A définie par la demi-coque principale 12A est généralement bombée vers l'extérieur afin de faciliter la préhension du corps 12 en reposant dans le creux de la main.

D'un même côté du conduit 14 et suivant un plan s'étendant généralement parallèlement à l'axe X-X du conduit, le corps 12 présente, dans sa surface latérale périphérique, deux creux successifs 52A, 52B destinés à former des appuis pour deux doigts consécutifs d'une main. Du côté opposé aux appuis-doigts 52A, 52B par rapport au conduit 14, le corps 12 présente, suivant sa surface latérale, une première et une deuxième surfaces convexes 53A, 53B adaptées chacune pour permettre l'appui de la paume de la main. Ces deux surfaces se rejoignent en un point d'inflexion 53C formé par le point du boîtier le plus éloigné du conduit 14.

Entre l'extrémité arrière 16 du conduit et les deux appuis-doigts 52A, 52B est délimité, dans le boîtier 12, un orifice 54 de passage du bouton poussoir 32. L'orifice 54 s'ouvre ainsi vers l'extérieur du côté de l'extrémité arrière 16.

En outre, au voisinage de l'extrémité avant 18 du conduit adaptée pour la liaison du cathéter, le corps 12 présente une troisième surface 56 formant appui-doigts. Cette surface 56 est ménagée, dans le mode de réalisation représenté, sur la surface latérale du piquage 22 et se trouve du même côté du conduit 14 que les creux 52A et 52B.

Comme illustré sur la figure 2, les moyens d'obturation 28 comportent un diaphragme constitué de deux joints accolés. Un premier joint 60 présente une surface concave tournée vers l'extrémité arrière 16 du conduit. Ce joint 16 est percé axialement d'un trou 62 normalement fermé lorsque le joint est au repos. Le second joint 64 présente trois entailles rectilignes s'étendant radialement et se rejoignant au centre du joint. Ces trois entailles sont décalées angulairement de 120° et définissent une étoile.

Le mécanisme de commutation 30 propre à modifier l'état des joints 60 et 64 comporte un équipage mobile 72. Ce dernier est déplaçable en translation parallèlement à l'axe X-X du conduit 14. Le mécanisme 30 comporte, en plus de l'équipage mobile 72, le bouton poussoir 32 et un ressort 74 de sollicitation du bouton poussoir vers une position de repos.

Plus précisément, l'équipage mobile 72 comporte un coulisseau 75 supportant une roue à rochet 76 montée rotative autour d'un axe Y-Y s'étendant parallèlement à l'axe X-X. Cette roue à rochet est adaptée pour coopérer avec des profils d'engrènement ménagés dans les demi-coques 12A et 12B. Ces profils seront décrits dans la suite de la description.

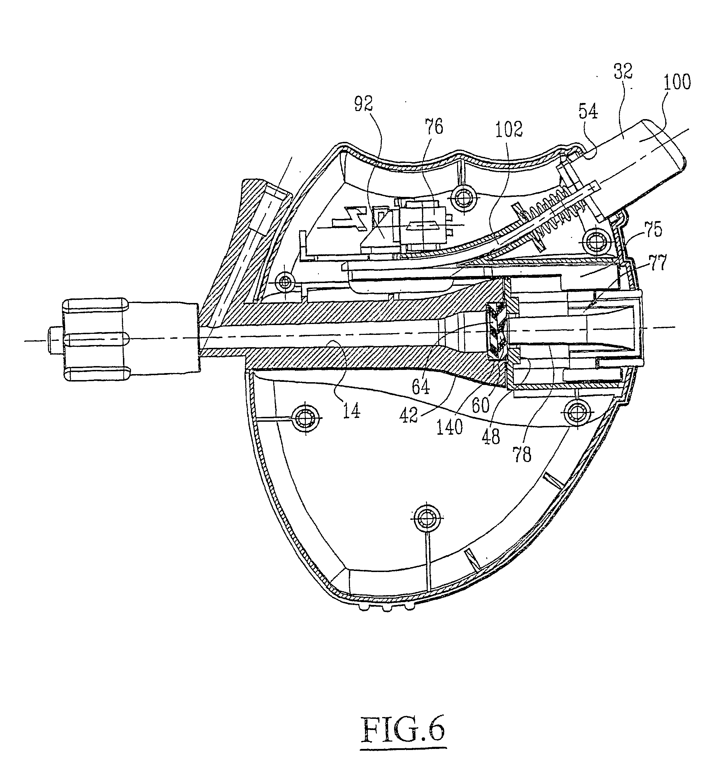

Le coulisseau 75 comporte, comme cela est visible également sur les figures 6 à 9, une canule 77 adaptée pour s'engager axialement à l'intérieur des joints 60 et 64 afin d'augmenter leur section de passage intérieur. Elle présente, en regard des joints, un manchon cylindrique 78 prolongé par un tronçon tronconique 80 s'évasant progressivement depuis le manchon 78. Ce tronçon tronconique 80 définit intérieurement une surface de guidage facilitant l'engagement des instruments opératoires dans le dispositif.

La canule 77 est bordée de part et d'autre, par deux flasques arqués 82 présentant des surfaces extérieures généralement cylindriques adaptées pour assurer un centrage par coopération avec la partie du conduit 14 définie par les tronçons semi-cylindriques 44 et 46.

En outre, de part et d'autre, les flasques 82 sont liés à des poutres de guidage 84, 86 adaptées pour s'engager dans des canaux rectilignes de guidage 88, 90 définis dans les deux demi-coques 12A, 12B, parallèlement à l'axe X-X du conduit 14. Ces poutres de guidage sont adaptées pour assurer un déplacement à coulissement de l'équipage mobile 72 par rapport au corps 12, et notamment par rapport au conduit 14 suivant la direction de l'axe X-X.

La poutre 86 se prolonge au-delà de la canule 77 jusqu'à une extrémité avant équipée d'une potence 92 adaptée pour supporter la roue à rochet 76. Cette potence présente une embase 94 supportant une broche 96 s'étendant suivant l'axe Y-Y et sur laquelle est engagée la roue à rochet 76 libre en rotation. La broche 96 présente une fente longitudinale et, à son extrémité libre, des ergots de retenue de la roue à rochet 76, laquelle est engagée par enclenchement élastique sur la broche 96.

Le bouton poussoir 32 présente une tête d'actionnement 100 de forme générale cylindrique adaptée pour coulisser au travers de l'orifice 54 et faire saillie hors du boîtier. Cette tête 100 est prolongée par une lame élastique 102 généralement courbe. La lame 102 présente, à son extrémité libre, une encoche 104 dans laquelle est engagée l'embase 96 de la potence, afin d'assurer une solidarisation de l'équipage mobile 72 et du bouton poussoir 32.

Les demi-coquilles 12A et 12B présentent chacune une rainure 106, 108 de guidage de la lame 102. Ces rainures sont généralement incurvées dans leur partie médiane afin de permettre des déplacements concomitants, d'une part, du bouton poussoir 32 suivant un axe Z-Z décalé angulairement par rapport à l'axe X-X du conduit 14 et, d'autre part, de l'extrémité libre de la lame reliée à l'équipage mobile 72 par l'encoche 104 suivant la direction de l'axe X-X.

L'angle défini entre les axes X-X et Z-Z est compris entre 20° et 60° et est par exemple de 30°.

Comme illustré sur la figure 6, le ressort 74 est un ressort de compression en spirale engagé autour de la lame élastique 102. Une extrémité du ressort s'appuie sur la tête 100 du bouton poussoir et dont l'autre extrémité prend appui sur une paroi 110 ménagée dans la demi-coque complémentaire 12B en regard de l'orifice 54.

La roue à rochet 76 est adaptée pour coopérer avec des surfaces de came portées par les deux demi-coques 12A, 12B afin de constituer un mécanisme de commande en va-et-vient de l'équipage mobile 72 à partir d'un mouvement unidirectionnel du bouton poussoir 32.

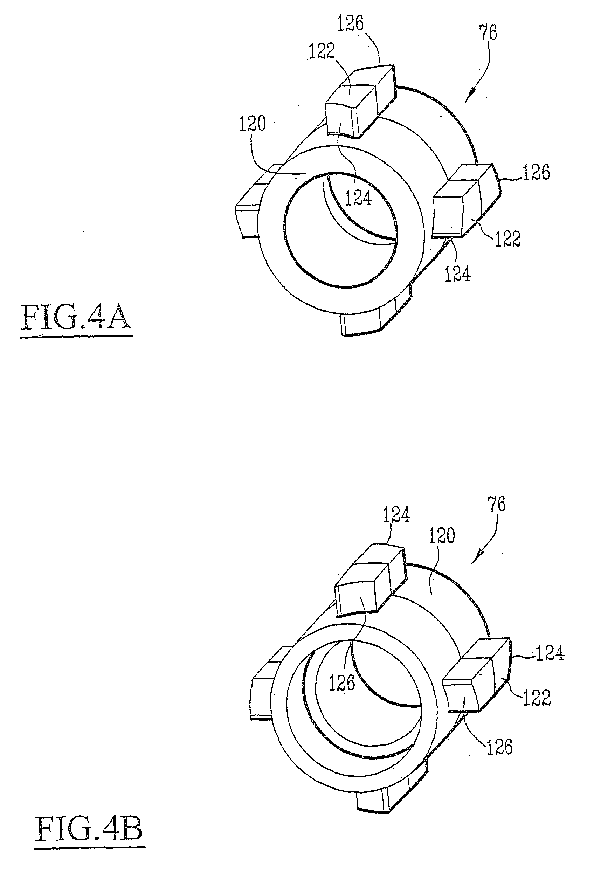

Plus précisément, la roue à rochet 76, représentée seule sur les figures 4A et 4B, est constituée d'un manchon tubulaire 120 sur la surface latérale duquel sont définies quatre nervures extérieures identiques 122.

Ces nervures 122 s'étendent suivant des génératrices du manchon 120 et sont décalées angulairement de 90°. Les faces d'extrémité des nervures 122 présentent chacune une rampe formant une surface de came.

Les surfaces de came sont généralement inclinées par rapport à un plan s'étendant perpendiculairement à l'axe du manchon 120 et convergent l'une vers l'autre dans le sens anti-horaire, lorsque les extrémités avant notées 124 des nervures sont tournées vers l'observateur, comme sur la figure 4A. Les extrémités avant des nervures sont celles disposées en regard de la base de la potence 96 lorsque la roue à rochet 76 est montée sur le coulisseau 74. Les extrémités arrière des nervures 120 sont notées 126. Elles sont visibles sur la figure 4B.

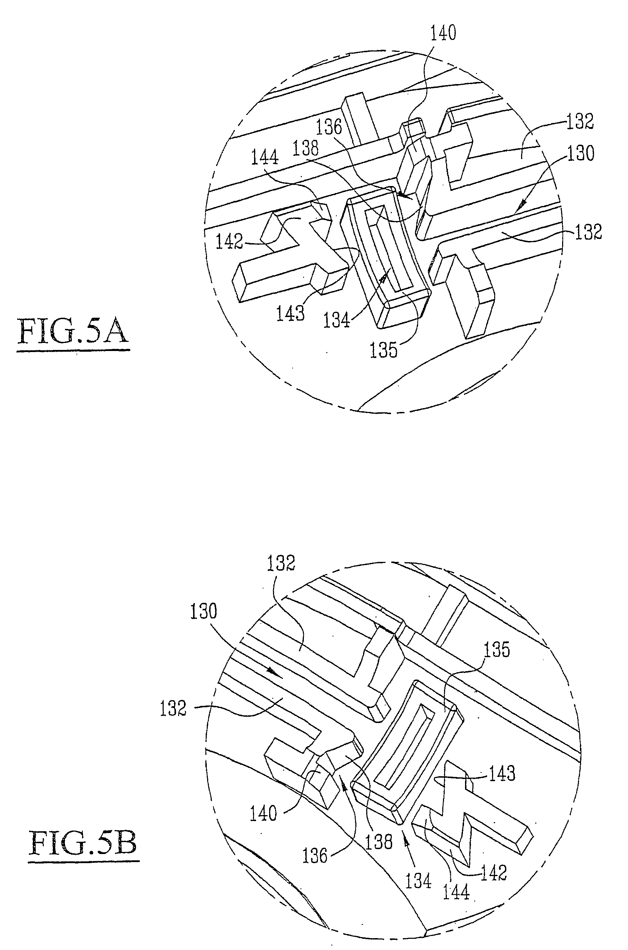

Les demi-coques 12A et 12B comportent, comme illustré sur les figures 5A et 5B, des reliefs adaptés pour coopérer avec les surfaces de came prévues aux extrémités 124 et 126 des nervures afin d'assurer l'entraînement en rotation de la roue à rochet 76, lors de l'enfoncement du bouton poussoir 32, ainsi que des reliefs adaptés pour assurer le maintien en position de la roue à rochet lors du relâchement du bouton poussoir 32. Les reliefs propres à assurer le déplacement angulaire de la roue à rochet sont adaptés pour entraîner celle-ci toujours dans un même sens.

Les reliefs des demi-coques 12A et 12B sont symétriques les uns des autres par rapport à l'axe Y-Y de rotation de la roue à rochet 76. Ces reliefs comportent, sur chaque demi-coque 12A, 12B, un canal 130 destiné à recevoir les nervures 122 de la roue à rochet et ainsi assurer une immobilisation en rotation de la roue, deux nervures 122 opposées étant alors retenues entre les rives parallèles notées 132 délimitant les canaux 130.

Chaque canal 130 est ouvert à une extrémité débouchant dans une plage 134 d'évolution en rotation de la roue à rochet. Cette région 134 présente un berceau 135 de support de la roue à rochet adaptée pour un guidage radial de celle-ci.

Au voisinage de l'extrémité débouchante du canal 130, une paroi de chaque demi-coque 12A, 12B présente une cavité 136 de réception de l'extrémité arrière 126 d'une nervure pour assurer l'immobilisation axiale et en rotation de la roue à rochet 76. Cette cavité 136 est bordée par une surface de guidage 138 reliant l'extrémité de l'une des rives 132 au fond de la cavité 136. Cette cavité 136 est adaptée pour la réception de l'extrémité arrière 126 d'une nervure et là retenue axiale de la roue à rochet et de l'ensemble de l'équipage mobile 72 dans une position telle que le dispositif est ouvert.

La cavité 136 est délimitée sur sa paroi opposée à la surface de guidage 138 par un pilier présentant, à son extrémité supérieure, une surface de came 140 propre à coopérer avec les surfaces de came des extrémités arrière 126 ménagées sur les nervures de la roue à rochet, afin de provoquer la rotation de cette roue à rochet d'un angle de 22,5°.

En outre, de l'autre côté de la plage 134 d'évolution de la roue à rochet, et sensiblement en regard de l'encoche 136, une saillie 142 de la demi-coque présente une première surface de came 143 sensiblement symétrique de la surface de came 138 et une seconde surface de came 144 sensiblement symétrique de la surface de came 140. Ces surfaces de came 143 et 144 sont adaptées chacune pour coopérer avec une surface de came ménagée à l'extrémité avant 124 de la roue à rochet afin de provoquer une rotation de la roue à rochet de 22,5°.

Lorsque le dispositif est assemblé, celui-ci est tel qu'illustré sur les figures 4 à 10. En particulier, les joints 60 et 64 sont confinés dans le conduit 14 en étant reçus dans un lamage 140 formé à l'extrémité du tronçon tronconique 42 obturé par le siège 48.

La canule 77 s'étend suivant l'axe du conduit 14 entre son extrémité arrière 16 et les joints 60 et 64. La tête 100 du bouton poussoir fait saillie hors du corps au travers de l'ouverture 54, alors que la lame 102 est engagée dans la glissière délimitée par les rainures 106 et 108. L'extrémité de la lame 102 présentant l'encoche 104 est engagée autour de l'embase 94. La roue à rochet 76 est portée par la broche 96 suivant l'axe Y-Y et s'étendant en regard des reliefs de guidage ménagés dans les deux demi-coques.

Sur la figure 6, le dispositif est dans sa position fermée. Dans cette position, la tête 100 du bouton poussoir fait saillie sur l'essentiel de sa hauteur hors du corps 12 et la canule 77 est disposée à l'écart des joints 60 et 64. Ces derniers n'étant pas sollicités, ils assurent une obturation complète du conduit 14 en l'absence de tout instrument médical engagé dans le conduit 14. Si un instrument médical est engagé, les joints 60 et 64 s'appliquent alors exactement autour de l'instrument médical les traversant, assurant ainsi une étanchéité évitant tout risque d'écoulement sanguin.

Dans cette position de l'équipage mobile 72 et du bouton poussoir 32, la roue à rochet 76 est disposée entre les canaux 130, deux nervures 122 opposées étant reçues dans ces canaux. La roue 76 est alors immobilisée en rotation.

Afin de permettre l'introduction d'un instrument médical dans le cathéter, les joints 60 et 64 doivent être ouverts. A cet effet, le bouton poussoir 32 est pressé suivant le sens de la flèche F1.

Lors de l'enfoncement du bouton poussoir 32, l'équipage mobile 72 est alors entraîné par la lame 102 en direction de l'extrémité avant du conduit 14. En particulier, la canule 77 pénètre au travers des joints 60 et 64, assurant ainsi une augmentation de la section offerte au passage dans le conduit 14.

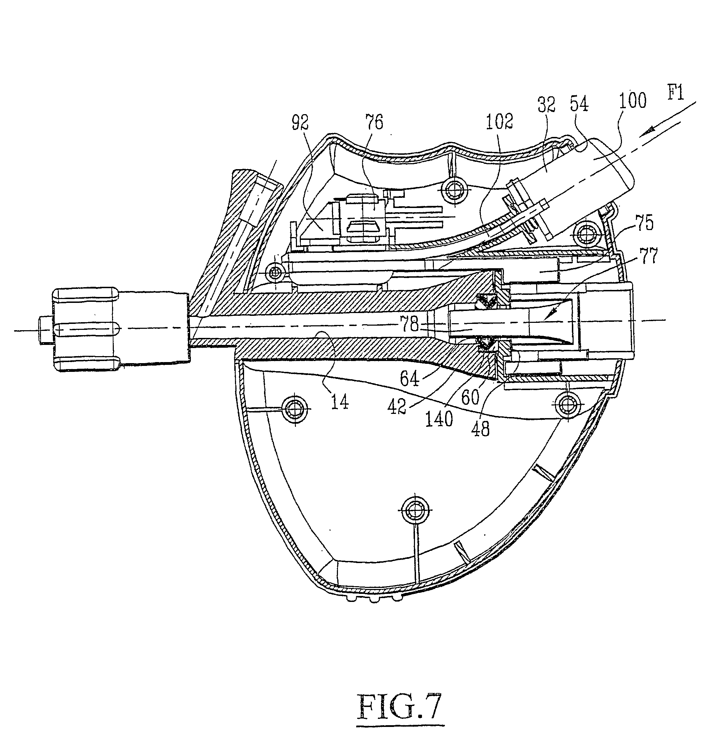

Par ailleurs, lors de l'enfoncement du bouton poussoir 32, la roue à rochet 76 est déplacée en translation vers l'avant suivant l'axe Y-Y, de sorte que les extrémités avant 124 des nervures non engagées dans les canaux 130 entrent en contact avec les surfaces de came 144 ménagées à l'avant de la région d'évolution 135 de la roue à rochet. Par effet de came, la roue à rochet est amenée à tourner de 22,5° pour être amenée dans la position de la figure 7.

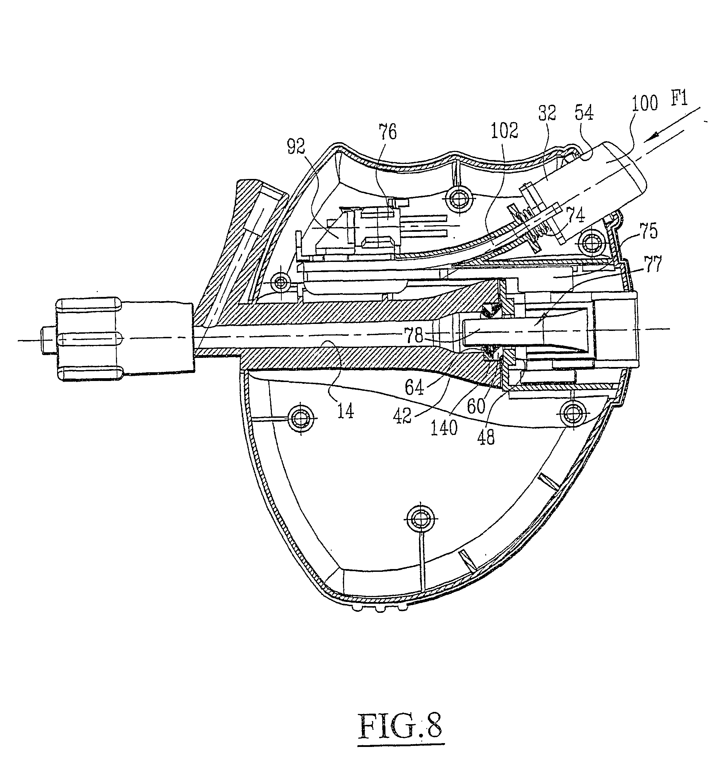

Le bouton poussoir 32 est alors relâché. Sous l'action du ressort 74, l'ensemble de l'équipage mobile 72 est ramené vers l'arrière. Lors de ce mouvement, les extrémités arrière des nervures opposées 122 coopèrent avec les rampes de guidage 138 afin d'assurer un déplacement angulaire de la roue à rochet 76 de 22,5° et d'amener l'extrémité arrière 126 de deux nervures opposées dans les cavités 136. Les nervures 122 étant en appui sur le fond des cavités 136 constituant des butées, la roue à rochet 76 ne peut poursuivre son déplacement en translation. Une immobilisation de l'équipage mobile 72 est alors obtenue dans la position représentée sur la figure 8, malgré l'action du ressort 74.

Le dispositif est alors dans une position stable et le conduit 14 est maintenu ouvert permettant l'introduction d'instruments médicaux au travers du conduit 14.

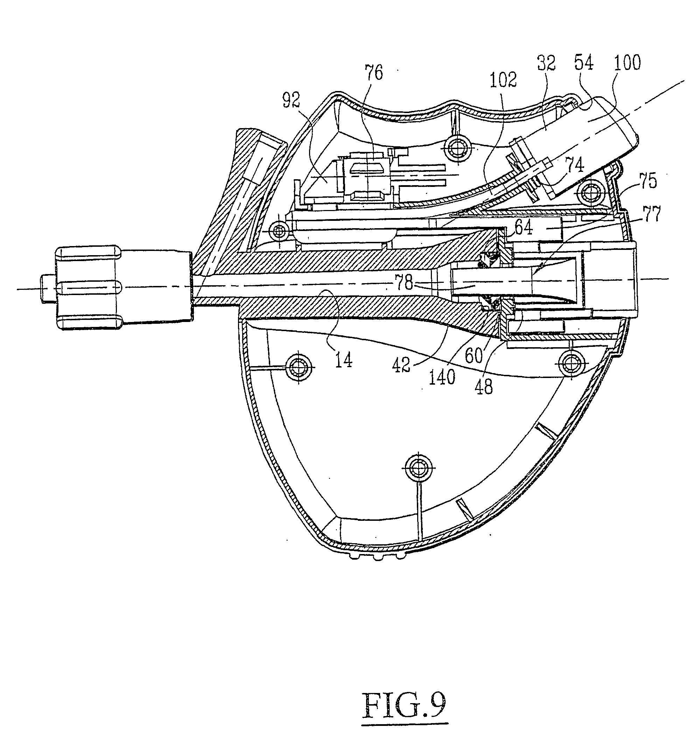

Afin de provoquer la fermeture du dispositif, le bouton poussoir 32 est à nouveau enfoncé dans la même direction et dans le même sens, c'est-à-dire suivant le sens de la flèche F1 que pour l'ouverture. L'équipage mobile 72 est alors déplacé vers l'avant du conduit 14 jusque dans la position illustrée sur la figure 9. Lors de son déplacement vers l'avant, la roue à rochet 76 entre en contact avec les surfaces de came 144 par l'intermédiaire des extrémités avant 124 de deux nervures opposées 122. Par effet de came, la roue à rochet est décalée angulairement de 22,5°.

Lors du relâchement ultérieur du bouton poussoir 32, les extrémités arrière 126 des nervures 122 portées par la roue à rochet entrent en contact avec les surfaces de came 140, provoquant alors par effet de came, la rotation supplémentaire de la bague d'un angle de 22,5°, de sorte que les nervures 122 se trouvent alignées avec les canaux 130. Sous l'action du ressort de rappel 74, les nervures opposées 122 se déplacent suivant la longueur des canaux 130, autorisant ainsi un retour vers l'arrière de l'équipage mobile jusque dans la position illustrée sur la figure 6. Dans cette position, la canule 77 est à l'écart des joints 60 et 64, assurant ainsi une obturation du conduit 14.

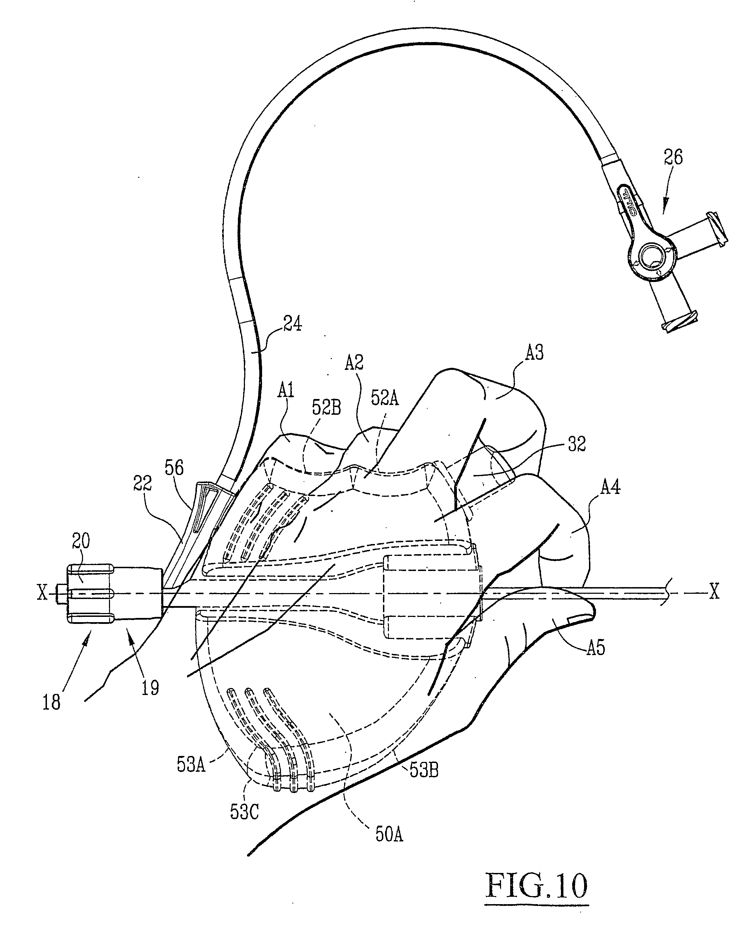

Sur les figures 10, 11 et 12 sont illustrés des modes de prise en main du dispositif correspondant à différents usages de celui-ci.

Plus précisément, sur la figure 10 est illustré le maniement du dispositif afin d'assurer l'introduction d'un instrument chirurgical filiforme dans le cathéter et son utilisation, lors d'une intervention d'angioplastie.

Pour cette utilisation, le dispositif est placé à plat dans le creux de la main avec sa surface plane 50A définie par la demi-coque 12A appliquée contre la surface intérieure de la main gauche.

Afin d'assurer une prise en main stable et une commande facile du bouton poussoir 32 et des instruments chirurgicaux introduits dans le cathéter, l'auriculaire A1 est placé sur l'appui-doigt 52B et l'annulaire A2 est placé sur l'appui-doigt 52B, de sorte que le majeur A3 s'étend immédiatement au-dessus du bouton poussoir 52 permettant un enfoncement et un relâchement facile de celui-ci. Les extrémités de l'auriculaire et l'annulaire sont appliquées sur la face bombée du boîtier définie par la demi-coque complémentaire 12B. L'index A4 et le pouce A5 s'étendent sensiblement en regard de l'extrémité arrière 16 du conduit 14 et permettent, comme illustré sur la figure 10, de maintenir entre eux un instrument filiforme K introduit ou en cours d'introduction dans le cathéter. Dans cette position, la surface d'appui 53A est appliquée sur la paume de la main, de sorte que le boîtier 12 est enserré entre l'auriculaire A1 et l'annulaire A2 d'un côté et la paume de la main de l'autre côté.

On comprend que, du fait de la forme du boîtier, et compte tenu de la position du bouton poussoir 32, le dispositif peut être manipulé simplement et commodément d'une seule main.

En particulier, le dispositif peut être utilisé facilement d'une seule main puisque l'actionnement du bouton 32 s'effectue toujours dans le même sens et suivant la même direction, que ce soit pour l'ouverture ou la fermeture du dispositif.

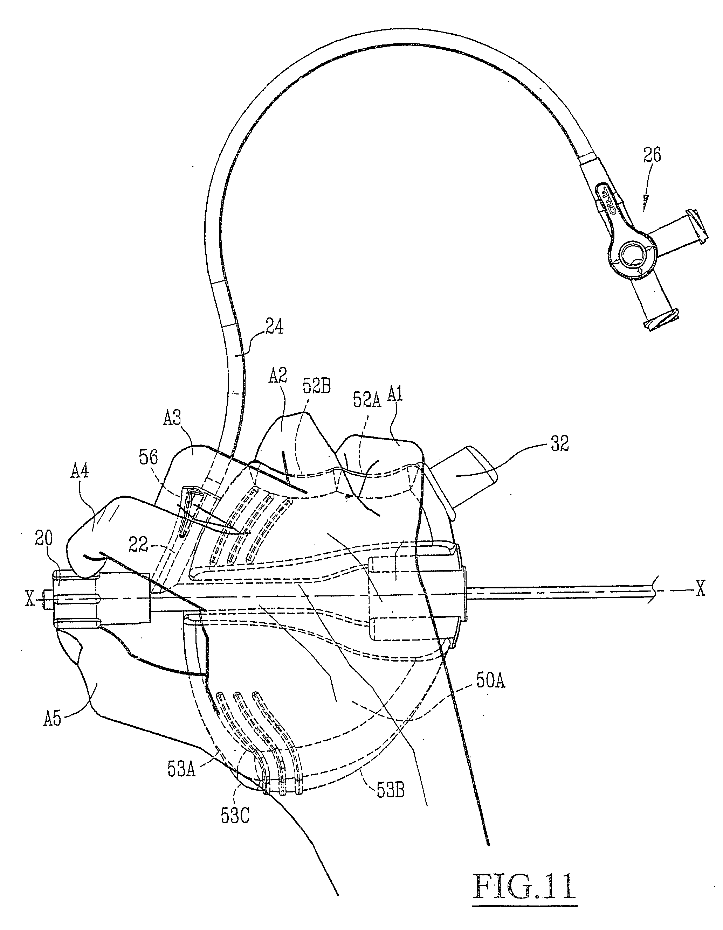

Sur la figure 11 est illustrée la manipulation du dispositif afin de permettre l'accouplement ou le désaccouplement du cathéter à l'extrémité avant 18 du conduit. Pour ce faire, le corps 12 du dispositif est maintenu dans la main droite de l'opérateur avec sa surface plane 50A définie par la demi-coque principale 12A en appui dans le creux de la main.

L'auriculaire A1 est appuyé sur l'appui-doigt 52A, l'annulaire A2 est appuyé sur l'appui-doigt 52B et le majeur A3 est appuyé sur l'appui-doigt 56. La bague 20 se trouve alors entre l'index A4 et le pouce A5. Ces deux derniers doigts formant pince permettent d'entraîner en rotation la bague 20 et ainsi d'accoupler ou désaccoupler le cathéter prolongeant le conduit 14. Dans cette position, la paume de la main s'appuie contre la surface d'appui 53B, de sorte que le corps 12 est retenu entre les trois doigts A1, A2, A3, d'une part, et la paume de la main, d'autre part.

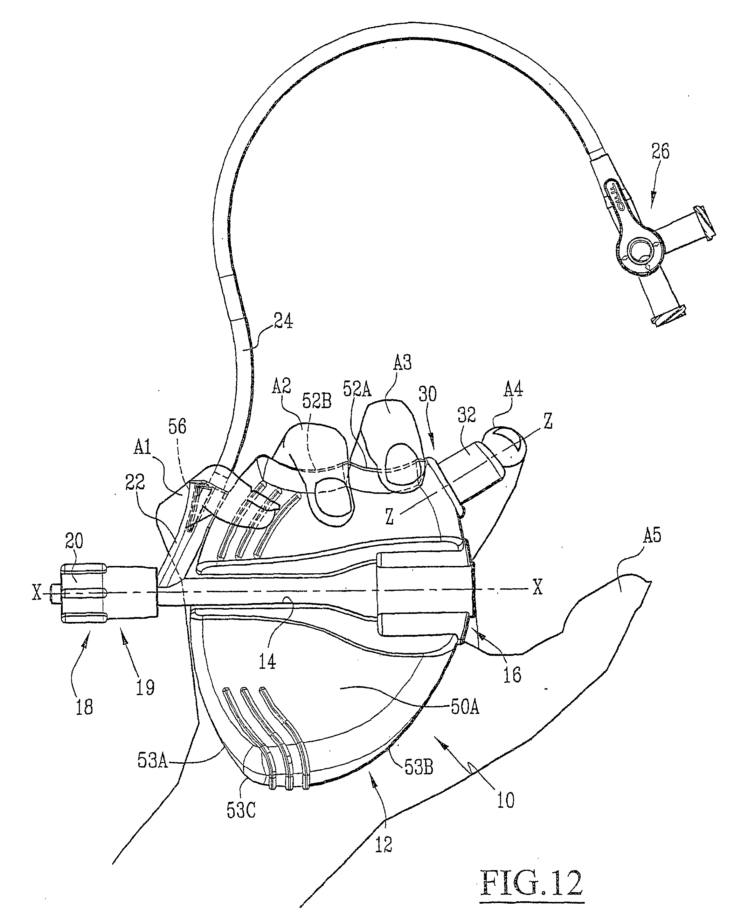

Sur la figure 12 est illustré un dernier mode de prise en main du dispositif permettant un "débullage" du cathéter relié au dispositif. L'opération de débullage consiste à extraire les bulles d'air éventuelles emprisonnées dans le cathéter, après mise en place de celui-ci. A cet effet, le conduit 14 doit être momentanément ouvert, sans qu'aucun instrument filiforme ne soit introduit par son extrémité arrière 16. Pour ce faire, le dispositif est saisi dans la main droite avec sa surface bombée 50B définie par la demi-coque supplémentaire 12B en appui dans le creux de la main.

Le corps 12 du dispositif est alors maintenu entre l'auriculaire A1, l'annulaire A2 et le majeur A3, d'un côté, et la paume de la main appliquée sur la surface d'appui 43B, de l'autre côté du conduit 14. Plus précisément, l'auriculaire A1 est appliqué sur l'appui-doigt 56, l'annulaire A2 est appliqué sur l'appui-doigt 52B et le majeur A3 est appliqué sur l'appui-doigt 52A. Les extrémités des trois doigts A1, A2 et A3 sont rabattues sur la surface bombée 50A du corps défini par la demi-coque principale 12A. L'index A4 s'étend alors immédiatement au droit du bouton poussoir 32 permettant une commutation aisée du dispositif entre son état ouvert et son état fermé afin d'assurer un débullage du cathéter. Lors d'une telle manipulation du dispositif, le pouce A5 est inutile et celui-ci est maintenu levé puis rabattu sur la surface plane de la demi-coque principale 12A.

Dans ces deux derniers modes de préhension du dispositif, on comprend également que les organes devant être manipulés, à savoir la bague 20 ou le bouton poussoir 32, sont immédiatement accessibles par le ou les doigts devant permettre la commande, alors que le corps du dispositif est maintenu fermement et de manière fiable dans la main du praticien.

On constate que quel que soit le mode d'utilisation du dispositif d'obturation, celui-ci peut être utilisé d'une seule main, libérant ainsi l'autre main du chirurgien.

## Revendications

1. Dispositif (10) d'obturation sélective de l'accès à l'intérieur d'un cathéter comportant, d'une part, un corps (12) délimitant un conduit (14) présentant une extrémité arrière (16) d'introduction d'un instrument médical et une extrémité avant (18) munie des moyens (19) de connexion au cathéter et, d'autre part, un mécanisme (27) de réglage de la section de passage offerte dans le conduit (14), lequel mécanisme de réglage (27) comporte un organe (32) de commutation du dispositif mobile par rapport au corps (12) suivant une course d'ouverture pour assurer la commutation vers un état ouvert, dans lequel une section de passage nominale est offerte dans le conduit (14), et suivant une course de fermeture pour assurer la commutation vers un état fermé, dans lequel le passage au travers du conduit (14) est au moins partiellement obturé, **caractérisé en ce que** le sens et la direction de déplacement de l'organe de commutation (32) suivant ladite course de fermeture, d'une part, et le sens et la direction de déplacement de l'organe de commutation suivant ladite course d'ouverture, d'autre part, sont identiques.

2. Dispositif selon la revendication 1, **caractérisé en ce que** la direction (Z-Z) de déplacement de l'organe de commutation (32) suivant lesdites courses d'ouverture et de fermeture est décalée angulairement par rapport à l'axe (X-X) du conduit (14).

3. Dispositif selon la revendication 1 ou 2, **caractérisé en ce que** ledit mécanisme (27) de réglage de la section de passage offerte dans le conduit (14) comporte un diaphragme (60, 64) et une canule (77) montée mobile dans le conduit (14) entre une position écartée du diaphragme (60, 64) correspondant à l'état fermé du dispositif, et une position engagée au travers du diaphragme (60, 64), correspondant à l'état ouvert du dispositif, et **en ce que** le mécanisme (27) comporte des moyens de déplacement de la canule (77) alternativement dans un sens et dans l'autre entre ses deux positions, sous la commande de l'organe de commutation (32), seulement lorsque l'organe de commutation (32) est déplacé, suivant lesdites courses d'ouverture et de fermeture, dans un sens de commande identique pour les deux sens de déplacement de la canule (77).

4. Dispositif selon l'une quelconque des revendications précédentes, **caractérisé en ce que** le conduit (14) est rectiligne, et **en ce que** ledit organe de commutation (32) est déplaçable en translation suivant lesdites courses d'ouverture et de fermeture parallèlement à l'axe (X-X) dudit conduit (14).

5. Dispositif selon la revendication 3 ou 4, **caractérisé en ce que** le mécanisme (27) de réglage de la section de passage offerte dans le conduit (14) comporte, d'une part, un coulisseau (75) dont la canule (77) est solidaire, lequel coulisseau (75) est déplaçable à coulissement par rapport au corps (12) sous la commande dudit organe de commutation (32) et, d'autre part, une roue à rochet (76) montée rotative par rapport au coulisseau (75) et fixe axialement par rapport à celui-ci, le corps (12) comportant des profils d'engrènement (144) de la roue à rochet (76) pour son déplacement angulaire lors du déplacement à coulissement du coulisseau, et des profils de guidage (138, 140) de la roue à rochet (76) pour son guidage lors du relâchement dudit organe de commutation (32) vers au moins deux positions axiales distinctes correspondant aux états ouvert et fermé du dispositif.

6. Dispositif selon l'une quelconque des revendications précédentes, **caractérisé en ce que** ledit mécanisme de commutation (27) comporte un organe (74) de rappel élastique de l'organe de commutation (32) suivant lesdites courses d'ouverture et de fermeture dans le sens opposé à celui produisant la commutation du dispositif.

7. Dispositif selon l'une quelconque des revendications précédentes, **caractérisé en ce que** le corps (12) présente une forme générale ovoïdale aplatie présentant deux surfaces principales opposées (50A, 50B) et une surface latérale périphérique reliant lesdites surfaces principales opposées (50A, 50B).

8. Dispositif selon la revendication 7, **caractérisé en ce que** le corps présente, sur sa surface latérale périphérique, au moins deux creux adjacents (52A, 52B) formant des-surfaces d'appui parmi les doigts d'une main.

9. Dispositif selon la revendication 8, **caractérisé en ce que** deux creux (52A, 52B) formant des surfaces d'appui pour les doigts d'une main sont disposés successivement suivant une direction sensiblement parallèle à l'axe (X-X) du conduit (14).

10. Dispositif selon la revendication 8, **caractérisé en ce que** ledit organe de commande (32) fait saillie hors du boîtier (12) entre deux creux (52A, 52B) formant des surfaces d'appui pour les doigts d'une main et l'extrémité arrière (16) du conduit (14).

11. Dispositif selon l'une quelconque des revendications précédentes, **caractérisé en ce qu'**il comporte des moyens (74, 136) de maintien du dispositif dans son état ouvert et dans son état fermé.

## Patentansprüche

1. Vorrichtung (10) für den selektiven Verschluß des Zugangs zum Inneren eines Katheters, mit, zum einen, einem Körper (12), der eine Rohrleitung (14) begrenzt, welche ein hinteres Ende (16) zur Einführung eines medizinischen Instruments und ein mit Mitteln (19) zur Verbindung mit dem Katheter versehenes vorderes Ende (18) aufweist, und, zum anderen, einem Einstellmechanismus (27) für den in der Rohrleitung (14) dargebotenen Durchlaßquerschnitt, welcher Einstellmechanismus (27) ein Umschaltorgan (32) der Vorrichtung aufweist, das in bezug auf den Körper (12) beweglich ist entlang eines Öffnungsweges zum Sicherstellen der Umschaltung in einen offenen Zustand, in welchem ein Nenn-Durchlaßquerschnitt in der Rohrleitung (14) dargeboten wird, und entlang eines Verschlußweges zum Sicherstellen der Umschaltung in einen geschlossenen Zustand, in welchem der Durchlaß durch die Rohrleitung (14) wenigstens teilweise verschlossen ist, **dadurch gekennzeichnet, daß** der Richtungssinn und die Richtung der Verstellung des Umschaltorgans (32) entlang des besagten Verschlußweges einerseits und der Richtungssinn und die Richtung der Verstellung des Umschaltorgans entlang des besagten Öffnungsweges andererseits identisch sind.

2. Vorrichtung nach Anspruch 1, **dadurch gekennzeichnet, daß** die Verstellrichtung (Z-Z) des Umschaltorgans (32) entlang der besagten Öffnungsund Verschlußwege winkelversetzt in bezug auf die Achse (X-X) der Rohrleitung (14) ist.

3. Vorrichtung nach Anspruch 1 oder 2, **dadurch gekennzeichnet, daß** der besagte Einstellmechanismus (27) für den in der Rohrleitung (14) dargebotenen Durchlaßquerschnitt eine Membran (60, 64) und eine Kanüle (77) aufweist, die in der Rohrleitung (14) beweglich zwischen einer Position abseits der Membran (60, 64), entsprechend dem geschlossenen Zustand der Vorrichtung, und einer Position, bei der sie quer durch die Membran (60, 64) gesteckt ist, entsprechend dem offenen Zustand der Vorrichtung, angebracht ist, und daß der Mechanismus (27) Mittel aufweist zum Verstellen der Kanüle (77) wechselweise in einem und dem anderen Verstellsinn zwischen ihren zwei Positionen, gesteuert durch das Umschaltorgan (32), nur dann, wenn das Umschaltorgan (32) entlang der besagten Öffnungs- und Verschlußwege in einem identischen Steuerungssinn für die zwei Verstellsinne der Kanüle (77) verstellt wird.

4. Vorrichtung nach einem der vorstehenden Ansprüche, **dadurch gekennzeichnet, daß** die Rohrleitung (14) geradlinig ist und daß das besagte Umschaltorgan (32) verstellbar ist in Verschiebung entlang besagter Öffnungsund Verschlußwege parallel zur Achse (X-X) der besagten Rohrleitung (14).

5. Vorrichtung nach Anspruch 3 oder 4, **dadurch gekennzeichnet, daß** der Einstellmechanismus (27) für den in der Rohrleitung (14) dargebotenen Durchlaßquerschnitt zum einen einen Schieber (75) aufweist, an dem die Kanüle (77) befestigt ist, welcher Schieber (75) gleitgeführt verstellbar in bezug auf den Körper (12) ist, gesteuert von besagtem Umschaltorgan (32), und, zum anderen, ein Stellrad (76), das drehbar in bezug auf den Schieber (75) und achsfest in bezug auf diesen montiert ist, wobei der Körper (12) Eingriffskonturen (144) für das Stellrad (76) für dessen Winkelverstellung bei der gleitgeführten Verstellung des Schiebers und Führungskonturen (138, 140) für das Stellrad (76) für dessen Führung bei der Freigabe des besagten Umschaltorgans (32) in wenigstens zwei axial unterschiedliche Positionen aufweist, welche den offenen und geschlossenen Zuständen der Vorrichtung entsprechen.

6. Vorrichtung nach einem der vorstehenden Ansprüche, **dadurch gekennzeichnet, daß** der besagte Umschaltmechanismus (27) ein Organ (74) aufweist für eine elastische Rückstellung des Umschaltorgans (32) entlang besagter Öffnungs- und Verschlußwege in entgegengesetztem Sinn zu dem die Umschaltung der Vorrichtung bewirkenden.

7. Vorrichtung nach einem der vorstehenden Ansprüche, **dadurch gekennzeichnet, daß** der Körper (12) eine allgemein eiförmige abgeflachte Form aufweist, die zwei gegenüberliegende Hauptoberflächen (50A, 50B) und eine seitliche Randoberfläche aufweist, die die besagten gegenüberliegenden Hauptoberflächen (50A, 50B) verbindet.

8. Vorrichtung nach Anspruch 7, **dadurch gekennzeichnet, daß** der Körper auf seiner seitlichen Randoberfläche wenigstens zwei benachbarte Mulden (52A, 52B) aufweist, die Auflageflächen unter den Fingern einer Hand bilden.

9. Vorrichtung nach Anspruch 8, **dadurch gekennzeichnet, daß** zwei Mulden (52A, 52B), welche Auflageflächen für die Finger einer Hand bilden, hintereinander entlang einer Richtung angeordnet sind, die im wesentlichen parallel zur Achse (X-X) der Rohrleitung (14) ist.

10. Vorrichtung nach Anspruch 8, **dadurch gekennzeichnet, daß** das besagte Steuerorgan (32) aus dem Gehäuse (12) zwischen zwei Auflageflächen für die Finger einer Hand bildenden Mulden (52A, 52B) und dem hinteren Ende (16) der Rohrleitung (14) hervorsteht.

11. Vorrichtung nach einem der vorstehenden Ansprüche, **dadurch gekennzeichnet, daß** sie Mittel (74, 136) zur Erhaltung der Vorrichtung in ihrem offenen und in ihrem geschlossenen Zustand aufweist.

## Claims

1. Device (10) for selectively blocking access to the inside of a catheter comprising, firstly, a body (12) defining a conduit (14) presenting a rear end (16) for inserting a medical instrument and a front end (18) provided with means (19) for connecting to the catheter and, secondly, a mechanism (27) for regulating the cross-section of passage offered in the conduit (14), the said regulating mechanism (27) comprising a member (32) for switching the device, movable relative to the body (12) along an opening travel to ensure switching to an open state, in which a nominal cross-section of passage is offered in the conduit (14), and along a closing travel to ensure switching to a closed state in which passage through the conduit (14) is at least partially blocked, **characterised in that**, firstly, the path and direction of displacement of the switching member (32) along the said closing travel and, secondly, the path and direction of displacement of the switching member along the said opening travel are identical.

2. Device according to claim 1, **characterised in that** the displacement direction (Z-Z) of the switching member (32) along the said opening and closing travels is angularly offset relative to the axis (X-X) of the conduit (14).

3. Device according to claim 1 or 2, **characterised in that** the said mechanism (27) for regulating the cross-section of passage offered in the conduit (14) comprises a diaphragm (60, 64) and a cannula (74) movably mounted in the conduit (14) between a position spaced from the diaphragm (60, 64), corresponding to the closed state of the device, and a position engaged through the diaphragm (60, 64), corresponding to the open state of the device, and **in that** the mechanism (27) comprises means for displacing the cannula (77) alternately in one direction and the other between its two positions, under the control of the switching member (32) only when the switching member (32) is displaced along the said opening and closing travels, in a direction of control which is identical for both displacement directions of the cannula (77).

4. Device according to any of the preceding claims, **characterised in that** the conduit (14) is rectilinear and **in that** the said switching member (32) is displaceable in translation along the said opening and closing travels parallel to the axis (X-X) of the said conduit (14).

5. Device according to claim 3 or 4, **characterised in that** the mechanism (27) for regulating the cross-section of passage offered in the conduit (14) comprises, firstly, a slide (75) integral with the cannula (77), the said slide (75) being slideably displaceable relative to the body (12) under the control of the said switching member (32) and, secondly, a ratchet wheel (76) rotatably mounted relative to the slide (75) and fixed axially relative to the latter, the body (12) comprising engaging sections (144) for the ratchet wheel (76) for its angular displacement during the sliding displacement of the slide, and guide sections (138, 140) for the ratchet wheel (76) to guide it when the said switching member (32) is released to at least two separate axial positions corresponding to the open and closed states of the device.

6. Device according to any of the preceding claims, **characterised in that** the said switching mechanism (27) comprises a resilient return member (74) for the switching member (32) along the said opening and closing travels in the opposite direction to that which causes the device to switch.

7. Device according to any of the preceding claims, **characterised in that** the body (12) is of generally flattened, oval shape having two opposed principal areas (50A, 50B) and a peripheral lateral surface connecting the said opposed principal surfaces (50A, 50B).

8. Device according to claim 7, **characterised in that** the body has, on its peripheral lateral surface, at least two adjacent hollows (52A, 52B) forming a seat for the fingers of one hand.

9. Device according to claim 8, **characterised in that** two hollows (52A, 52B) forming seats for the fingers of one hand are successively arranged in a direction which is substantially parallel to the axis (X-X) of the conduit (14).

10. Device according to claim 8, **characterised in that** the said control member (32) projects outside the casing (12) between two hollows (52A, 52B) forming seats for the fingers of one hand and the rear end (16) of the conduit (14).

11. Device according to any of the preceding claims, **characterised in that** it comprises means (74, 136) for maintaining the device in its open state and its closed state.
